Europäisches Patentamt

European Patent Office

Office européen des brevets

(11) Veröffentlichungsnummer: **0 235 535**
**B1**

# EUROPÄISCHE PATENTSCHRIFT

(45) Veröffentlichungstag der Patentschrift:
02.08.89

(51) Int. Cl.⁴: **C07C 19/00, C07C 17/33**

(21) Anmeldenummer: **87100830.6**

(22) Anmeldetag: **22.01.87**

(54) Verfahren zur Herstellung von Hexachlorethan aus Hexachlorbutadien.

(30) Priorität: 01.03.86 DE 3606746

(43) Veröffentlichungstag der Anmeldung:
09.09.87 Patentblatt 87/37

(45) Bekanntmachung des Hinweises auf die Patenterteilung:
02.08.89 Patentblatt 89/31

(84) Benannte Vertragsstaaten:
BE DE ES FR GB IT NL SE

(56) Entgegenhaltungen:
DE-C- 801 987

CHEMICAL ABSTRACTS, Band 70, Nr. 3, 20.
Januar 1969, Seite 221, Zusammenfassung Nr. 11103g,
Columbus, Ohio, US; & JP-A-68 04 326
CHEMICAL ABSTRACTS, Band 89, Nr. 15, 9.
Oktober 1978, Seite 548, Zusammenfassung Nr. 129041f,
Columbus, Ohio, US; & JP-A-78 65 804

(73) Patentinhaber: HÜLS AKTIENGESELLSCHAFT,
Patentabteilung / PB 15 - Postfach 13 20,
D-4370 Marl 1(DE)

(72) Erfinder: Sticken, Gerhard, Dr., Am Gecksbach 32,
D-4270 Dorsten 11(DE)

**Beschreibung**

Hexachlorbutadien fällt bei allen Chlorolyseverfahren, d. h. bei allen Hochtemperaturchlorierungen von Kohlenwasserstoffen und Chlorkohlenwasserstoffen zu Perchlorethylen und Tetrachlorkohlenstoff an. Bei den eingesetzten Chlorkohlenwasserstoffen handelt es sich zumeist um Abfallprodukte aus anderen Chlorierungsprozessen. Der Anfall von Hexachlorbutadien, bezogen auf die Produktion von Perchlorethylen + Tetrachlorkohlenstoff, liegt bei etwa 5 Gewichtsprozent (Ullmanns Encyklopädie der technischen Chemie, 4. Auflage, Band 9, Seite 461 und 475, Verlag Chemie, Weinheim, 1975).

Wegen der derzeitigen hohen Kapazitäten der Chlorolyseanlagen fällt allein hier mehr Hexachlorbutadien an, als benötigt wird, wobei die Anwendungsgbiete ohnehin beschränkt sind. Hexachlorbutadien findet beispielsweise als Absorptionsmittel zur Entfernung von Verunreinigungen aus Gasen und als Kühlmittel in Transformatoren Anwendung. Somit muß überschüssiges Hexachlorbutadien entsorgt werden über Deponie, Verbrennung bzw. Rückführung in die Chlorolyseprozesse. Alle diese Maßnahmen sind aufwendig und teuer. Die Rückführung von Hexachlorbutadien in Chlorolyseprozesse stellt zudem keine Ideallösung dar, weil hierbei aus Hexachlorbutadien erhebliche Mengen Hexachlorbenzol entstehen, die ihrerseits entsorgt werden müssen.

Es ist in den letzten Jahren versucht worden, Hexachlorbutadien durch gezielte chemische Umsetzungen in verwertbare Substanzen zu überführen. Zwei japanische Patentanmeldungen (s. unten) schildern genau diese Situation und bieten als Lösungsweg die UV-Licht-katalysierte Chlorierung von Hexachlorbutadien zu Hexachlorethan an. Dieser Literatur zufolge ist Hexachlorethan u. a. als Einsatzstoff zur Herstellung wichtiger Fluorchlorkohlenwasserstoffe wie z. B. Trifluortrichlorethan (F 112) einsetzbar; außerdem soll es zur Herstellung von Nebelkerzen und Rauchbomben verwendet werden.

Schließlich kann Hexachlorethan relativ einfach durch Chlorabspaltung in das breit angewandte Lösemittel Perchlorethylen überführt werden. Betreiber von Chlorolyseanlagen können das Hexachlorethan zu diesem Zwecke direkt dem Einsatzgemisch zugeben, so wie es mit dem bei der Chlorolyse anfallenden Hexachlorethan als Rückführung schon praktiziert wird (Ullmanns Encyklopädie der technischen Chemie, 4. Auflage, Band 9, Seite 462, Verlag Chemie, Weinheim, 1975).

Während die JP-OS 49/000 208 der Firma Toa Goṣei Chem. Ind. die Herstellung von Hexachlorethan aus Hexachlorbutadien unter UV-Licht-Bestrahlung in Gegenwart von Tetrachlorkohlenstoff als Lösemittel umfaßt, verzichtet das Verfahren nach der japanischen Offenlegungsschrift JA-OS 53/065 804 der Sumitomo Chemical auf das Lösemittel und wendet statt dessen einen leicht erhöhten Chlordruck an. Die Reaktionstemperatur soll bei letzterem Verfahren zwischen 70 und 100 °C liegen, bei ersterem zwischen 0 und 200 °C, vorzugsweise zwischen 50 und 100 °C. Ziel ist in beiden Fällen die weitgehende Vermeidung von "Hochsiederbildungen", d. h. insbesondere die Vermeidung der Bildung des unerwünschten Nebenproduktes Decachlorbutan.

In beiden Fällen werden zur näheren Erläuterung der erfindungsgemäßen Verfahren Beispiele von Versuchen im Labormaßstab angegeben. Zur Übertragbarkeit in technische Größenordnungen werden keine Angaben gemacht. Eigene Versuche hierzu zeigten nun, daß gerade an dieser Stelle bei beiden JP-Verfahren Probleme auftreten, da die Eindringtiefe von UV-Licht in das Reaktionsmedium sehr gering ist. Wollte man einigermaßen akzeptable Reaktionsgeschwindigkeiten erzielen, so müßte eine möglichst große Oberfläche zwischen UV-Licht-Strahler und Reaktionsmedium geschaffen werden, d. h. man müßte die derzeit größten verfügbaren UV-Licht-Strahler (einige 10 kW) einsetzen. Die Energie derartiger Strahler würde dabei jedoch nur zu einem Bruchteil ausgenutzt werden. Kosten und erreichbare Raum-Zeit-Ausbeuten stünden in einem sehr ungünstigen Verhältnis!

Auf die UV-Licht-Bestrahlung zu verzichten und statt dessen mit Eisen(III)chlorid als Katalysator bei 100 bis 140 °C zu arbeiten, wie es in der russischen Patentschrift SU-PS 283 204 beschrieben wird, erscheint auch nicht empfehlenswert: Für eine Reaktionszeit von 20 Stunden wird eine Ausbeute von 54 % Hexachlorethan angegeben. Über den Umsatz von Hexachlorbutadien im gleichen Zeitraum wird nichts ausgesagt. Nach einer Arbeit von A. Roedig (Annalen der Chemie 574 (1951), Seite 122 ff) dürften bei dieser Verfahrensweise jedoch erhebliche Mengen Decachlorbutan und/oder Octachlorbuten als Nebenprodukte entstehen, für welche keine Verwendung besteht und die demzufolge entsorgt werden müßten.

Da in der SU-Patentschrift Angaben zum Umsatz bzw. zur Bildung von Nebenprodukten fehlen, entzieht sich das Verfahren einer sachkundigen Beurteilung.

Es wurde nun überraschenderweise ein für die technische Realisierung geeignetes Verfahren gefunden, welches den Vorteil der UV-Licht-katalysierten Chlorierung von Hexachlorbutadien zu Hexachlorethan hinsichtlich einer minimalen Nebenproduktbildung aufweist, den Nachteil dieser Vorgehensweise hinsichtlich der geringen Raum-Zeit-Ausbeute jedoch überwindet und welches sich gegenüber der Fe- und/oder FeCl₃-katalysierten Chlorierung gemäß der russischen Patentschrift hinsichtlich Raum-Zeit-Ausbeute und Nebenproduktbildung vorteilhaft abhebt.

Das erfindungsgemäße Verfahren ist dadurch gekennzeichnet, daß die Chlorierung von Hexachlorbutadien zu Hexachlorethan in Gegenwart von Sauerstoff durchgeführt wird.

Zweckmäßigerweise wird der Sauerstoff gasförmig dem ebenfalls gasförmigen Chlorstrom zudosiert. Die vollständige Mischung der Gasströme gewährleistet einen stationären Reaktionsablauf nach Einleiten in den flüssig vorgelegten Reaktionspartner Hexachlorbutadien.

Die Vermischung des gasförmigen Chlor-Sauerstoff-Stromes mit dem flüssigen Hexachlorbutadien kann sowohl durch den Gasstrom selbst als auch durch Rührwerke vorgenommen werden. Für den erstgenannten Fall wählt man zweckmäßigerweise als Reaktor eine Blasensäule. Zum Betreiben der Blasensäule muß Chlor im Überschuß eingesetzt werden.

Es hat sich gezeigt, das lineare Gasgeschwindigkeiten in der Blasensäule von etwa 0,3 bis 1,0 cm/s für eine gute Durchmischung ausreichen. Höhere lineare Gasgeschwindigkeiten bewirken im allgemeinen keinen verbesserten Reaktionsablauf.

Erfindungsgemäß wird die Umsetzung von Hexachlorbutadien zu Hexachlorethan durch Sauerstoff günstig beeinflußt; die Nebenproduktbildung ist gering, die Raum-Zeit-Ausbeute groß, was für die Wirtschaftlichkeit des Verfahrens von wesentlicher Bedeutung ist.

Hinsichtlich der für das Verfahren erforderlichen Sauerstoffmengen ist festzustellen, daß die Umsatzgeschwindigkeit des Hexachlorbutadiens durch Erhöhung des Sauerstoffgehaltes im Einsatz-Chlor-Sauerstoff-Strom erheblich gesteigert werden kann.

Im unteren Konzentrationsbereich zwischen 0 und etwa 1,0 Vol.-% Sauerstoff nimmt die Umsatzgeschwindigkeit des Hexachlorbutadiens mit steigendem Sauerstoffgehalt zunächst nur geringfügig zu. Dennoch ist auch dieser Bereich für die technische Anwendung geeignet. Beispiel 5 zeigt, daß in Anwesenheit von nur 1 Vol.-% Sauerstoff (entsprechend 5 Vol.-% Luft) die Reaktion bereits mit technisch akzeptabler Geschwindigkeit abläuft.

Demgegenüber wird die Reaktion in Abwesenheit von Sauerstoff nach vergleichbar raschem Start bereits nach 10 bis 20 % Hexachlorbutadienumsatz deutlich langsamer und kommt nach 25 bis 30 % Hexachlorbutadienumsatz praktisch ganz zum Erliegen, wie unmittelbar dem Vergleichsbeispiel zu entnehmen ist.

Eine ausgeprägte Zunahme der Hexachlorbutadienumsatzgeschwindigkeit mit steigendem Sauerstoffanteil im Einsatz-Chlor-Sauerstoff-Strom ist bei Sauerstoffanteilen größer 1 Vol.-% zu beobachten (Beispiele 3 und 2).

Mit weiter zunehmenden Sauerstoffanteilen verringert sich jedoch der in den Beispielen offenbarte Effekt (s. Beispiele 2 und 7). Schließlich bringt eine vermehrte Sauerstoffzugabe keine weitere Steigerung der Umsatzgeschwindigkeit. Es hat sich gezeigt, daß Sauerstoffgehalte im Einsatz-Chlor-Sauerstoff-Strom größer 10 Vol.-% keine bemerkenswerten Steigerungen mehr bewirken.

Ein weiterer wesentlicher Parameter für das erfindungsgemäße Verfahren ist der Gesamtdruck.

Es hat sich gezeigt, daß die Erhöhung des Gesamtdruckes im Reaktionssystem in jedem Fall eine Beschleunigung der Chlorierung des Hexachlorbutadiens zu Hexachlorethan bewirkt. Die Beispiele 1 und 2 zeigen den Effekt für die Steigerung des Gesamtdruckes von 1,0 auf 4,0 bar (abs.) bei einem Sauerstoffgehalt von 5,0 Vol.-% im Einsatz-Chlor-Sauerstoff-Strom und bei einer Reaktionstemperatur von 140 °C.

Da Chlor in technischen Anlagen im allgemeinen bis zu einem Druck von etwa 10 bar (abs.) verfügbar ist, empfiehlt es sich, diesen Druck so weit wie möglich zu nutzen. Es ist aber auch möglich, mit deutlich höherem Gesamtdruck zu arbeiten, sofern die technischen Gegebenheiten das zulassen. Die obere Grenze des zu wählenden Gesamtdruckes ist der Wert, der zur Verflüssigung des Chlors führen würde. Die Verwendung von flüssigem Chlor ist unzweckmäßig, weil sie, um einen gleichmäßigen Reaktionsablauf zu gewährleisten, erhebliche Anforderungen an die Vermischung der Reaktionspartner stellt. In der Regel sind Gesamtdrücke bis etwa 50 bar (abs.) praktikabel.

Die Reaktionstemperatur ist ebenfalls ein verfahrenskritischer Parameter.

Es hat sich gezeigt, daß in Bereichen von 140 bis 180 °C kaum Änderungen der Umsatzgeschwindigkeit beobachtet werden, wenn bei mäßigen Gesamtdrücken (1 bis 4 bar (abs.) gearbeitet wird (Beispiele 4 und 5). Unter 100 °C Reaktionstemperatur nimmt die Umsatzgeschwindigkeit dagegen stark ab; zudem werden längere Induktionsperioden (Zeiten bis zum Einsetzen der Reaktion) beobachtet (einige Stunden, s. Beispiel 6). Eine Anhebung der Reaktionstemperatur auf über 180 °C ist grundsätzlich möglich, kann aber entsprechend der Zunahme des Eigendampfdruckes des Hexachlorbutadiens, dessen Siedepunkt bei Normaldruck bei 216 °C liegt, zu einem relativ starken Abfall des Chlorpartialdruckes und damit zu einer Abnahme der Umsatzgeschwindigkeit führen.

Als Sauerstofflieferant für das erfindungsgemäße Verfahren ist neben reinem Sauerstoff beispielsweise auch Luft geeignet. Der Stickstoffanteil der Luft bewirkt jedoch ebenfalls eine Verringerung des Chlorpartialdruckes und damit eine Verringerung der Reaktionsgeschwindigkeit. Dennoch kann Luft anstelle von reinem Sauerstoff eingesetzt werden, wenn vorerwähnter Nachteil gering ist, in Kauf genommen oder durch die Wahl anderer Parameter ausgeglichen werden kann. Die Beispiele 3 und 5 zeigen, daß es bei einem Gesamtdruck von 4 bar (abs.) und einer Reaktionstemperatur von 140 °C für die Umsatzgeschwindigkeit des Hexachlorbutadiens praktisch unerheblich ist, ob dem Chloreinsatzstrom 1,0 Vol.-% Sauerstoff in Form von reinem Sauerstoff oder in Form von Luft (5,0 Vol.-%) zudosiert wird.

Die erfindungsgemäße Chlorierung von Hexachlorbutadien zu Hexachlorethan kann auch in Gegenwart eines Lösemittels wie z. B. Tetrachlorkohlenstoff durchgeführt werden. Es ist allerdings zu berücksichtigen, daß der Partialdruck des Lösemittels eine Verringerung des Chlorpartialdruckes und damit eine Verringerung der Reaktionsgeschwindigkeit bewirkt.

Bei der Durchführung des erfindungsgemäßen Verfahrens ist ferner zu beachten, daß Eisen bzw. Eisenverbindungen stören können. In der Flüssigphase sind allenfalls Eisen(III)chloridanteile bis etwa 2 Gew.-ppm tolerierbar; bei einem Gehalt von 10 bis 40 Gew.-ppm kommt die Chlorierungsreaktion fast zum Erliegen; bei Gehalten oberhalb 50 Gew.-ppm Eisen(III)chlorid nimmt zwar die Hexachlorbutadienumsatzgeschwindigkeit wieder zu, es entsteht jedoch nunmehr überwiegend Octachlorbuten als Reaktionsprodukt. Eine zumindest teilweise Aufhebung dieser Beeinträchtigung des Reaktionsgeschehens gelingt durch Zugabe geringer Wassermengen, z. B. mit dem Gas-Einsatz-Strom.

Eine Beeinträchtigung des Hexachlorbutadienumsatzes kann schließlich auch durch anwesendes Brom und/oder durch bromorganische Verbindungen eintreten; insbesondere können längere Induktionsperioden auftreten.

In technischem Hexachlorbutadien möglicherweise anwesendes Perchlorethylen und/oder Hexachlorethan führt zu keinen nennenswerten Störungen der Chlorierungsreaktion.

Wird das erfindungsgemäße Verfahren mit Chlorüberschuß betrieben, z. B. in einer Blasensäule als Reaktor, erhält man einen Chlor-Sauerstoff-Strom als Abgas mit gegebenenfalls zusätzlich anwesenden Inerten. Ist der Inertgasanteil gering, kann dieser Strom praktisch vollständig unter Ergänzung des umgesetzten Chlors in die Reaktion zurückgeführt werden. Müssen größere Inertgasmengen ausgeschleust werden, empfiehlt es sich, daraus den Chloranteil mit einem Lösemittel (z. B. Tetrachlorkohlenstoff) auszuwaschen. Nach Ausdampfen aus dem Lösemittel kann dieser Chloranteil dem Reaktionssystem wieder zugeführt oder auch an anderer Stelle nutzbringend eingesetzt werden.

Das erfindungsgemäße Verfahren kann kontinuierlich und diskontinuierlich durchgeführt werden.

Die nachfolgenden Beispiele erläutern das erfindungsgemäße Verfahren, ohne es darauf zu beschränken.

## Beispiel 1

3 kg Hexachlorbutadien werden bei 140 (+ 5) °C mit 300 Nl Chlor pro Stunde in einem Blasensäulenreaktor (Durchmesser: 100 mm) begast. Der Sauerstoffgehalt des Chlor-Sauerstoff-Einsatz-Stromes beträgt 5,0 Vol.-%, der Druck im Reaktionssystem 1,0 bar (abs.).
Die Reaktion setzt nach einer Induktionsperiode von etwa 45 Minuten ein. Nach insgesamt 3,0 Stunden sind 10,7 % Hexachlorbutadien umgesetzt worden, davon 76 % zu Hexachlorethan.
Nach Einsetzen der Reaktion werden rund 10 % der angebotenen Chlormenge umgesetzt, der Rest verläßt unumgesetzt das System.

## Beispiel 2

Die Versuchsanordnung nach Beispiel 1 wird unter sonst gleichen Bedingungen bei einem Druck von 4,0 bar (abs.) betrieben.
Die Reaktion setzt nach einer Induktionsperiode von etwa 20 Minuten ein. Nach insgesamt 2,0 Stunden sind 50,7 % Hexachlorbutadien umgesetzt worden, davon 83 % zu Hexachlorethan.
Nach Einsetzen der Reaktion werden rund 70 % der angebotenen Chlormenge umgesetzt, der Rest verläßt unumgesetzt das System.

## Beispiel 3

Die Versuchsanordnung nach Beispiel 2 wird unter sonst gleichen Bedingungen mit einem Chlor-Sauerstoff-Einsatz-Strom betrieben, wobei der Sauerstoffgehalt gegenüber Beispiel 2 von 5,0 auf 1,0 Vol.-% abgesenkt worden ist.
Die Reaktion setzt nach einer Induktionsperiode von etwa 30 Minuten ein. Nach insgesamt 2,0 Stunden sind 14,6 % Hexachlorbutadien umgesetzt worden, davon 80 % zu Hexachlorethan.

## Beispiel 4

3 kg Hexachlorbutadien werden bei 180 °C und 4,0 bar (abs.) mit 350 Nl Chlor pro Stunde in einem Blasensäulenreaktor begast. Der Chlorgasstrom enthält 5,0 Vol.-% Luft als Sauerstofflieferanten.
Die Reaktion startet nach einer Induktionsperiode von rund 15 Minuten. Nach 2, 3, 4, 5 Stunden sind 15, 22, 30, 37 % Hexachlorbutadien umgesetzt worden, davon jeweils 88 % zu Hexachlorethan.

## Beispiel 5

Die Versuchsanordnung nach Beispiel 4 wird bei einer Temperatur von 140 °C und sonst gleichen Bedingungen betrieben.
Die Induktionsperiode dauerte wie in Beispiel 4 rund 15 Minuten. Nach 2, 3, 4, 5 Stunden sind 17, 26, 35, 43 % Hexachlorbutadien umgesetzt worden, davon jeweils 85 % zu Hexachlorethan.

## Beispiel 6

Die Versuchsanordnung nach Beispiel 4 wird unter sonst gleichen Bedingungen bei einer Temperatur von 100 °C betrieben.
Die Reaktion startet nach einer Induktionsperiode von etwa 2 Stunden.
Nach insgesamt 5 Stunden sind 5,7 % Hexachlorbutadien umgesetzt worden, davon rund 20 % zu Hexachlorethan.

## Beispiel 7

Die Versuchsanordnung nach Beispiel 2 wird unter sonst gleichen Bedingungen mit einem leicht erhöhten Chlor-Sauerstoff-Einsatz-Strom von 390 Nl pro Stunde betrieben, wobei der Sauerstoffgehalt gegenüber Beispiel 2 von 5,0 auf 8,9 Vol.-% angehoben worden ist.
Die Reaktion setzt nach einer Induktionsperiode von etwa 10 Minuten ein. Nach insgesamt 2,0 Stunden Reaktionszeit sind 64,0 %Hexachlorbutadien umgesetzt worden, davon 78 % zu Hexachlorethan.

## Beispiel 8

Ein Blasensäulenreaktor von 324 mm innerem Durchmesser wird mit 23 l eines Gemisches Tetrachlorkohlenstoff-Hexachlorbutadien im Gewichtsverhältnis 3 zu 1 gefüllt. Das hier verwendete, technische Hexachlor butadien ist einem Chlorolyseprozeß entnommen; es enthält 8 Gew.-%

Hexachlorethan und 11 Gew.-% Perchlorethylen.

Bei 140 °C und 4 bar (abs.) werden zunächst 11 Stunden lang 1500 Nm³ Chlor pro Stunde durchgesetzt. Der Sauerstoffanteil beträgt 5,0 Vol.-%.

Anschließend wird der Blasensäulenreaktor mit 4,1 kg pro Stunde (etwa 3 l pro Stunde) Flüssigphase der vorgenannten Zusammensetzung kontinuierlich beaufschlagt.

Nach etwa 40 Stunden kontinuierlichem Betrieb haben sich stationäre Reaktionsverhältnisse eingestellt. Es werden 40 % Hexachlorbutadien umgesetzt, davon 82 % zu Hexachlorethan.

Das im Einsatz befindliche Perchlorethylen ist im Austrag nicht mehr nachweisbar; es ist vollständig in Hexachlorethan übergeführt worden.

Vergleichsbeispiel

Die Versuchsanordnung nach Beispiel 5 wird unter sonst gleichen Bedingungen ohne jegliche Sauerstoffzugabe betrieben (Reinheit des Einsatzchlors: 99,99 Vol.-%).

Die Reaktion startet nach einer Induktionsperiode von etwa 20 Minuten. Nach einer Zeit von insgesamt 2,0 Stunden sind rund 17 % Hexachlorbutadien umgesetzt worden, davon rund 75 % zu Hexachlorethan. Das entspricht etwa dem auch in Anwesenheit von Sauerstoff erzielten Umsatz, woraus zu entnehmen ist, daß die Anfangsgeschwindigkeit des Hexachlorbutadienumsatzes zu Hexachlorethan bis zu diesem Zeitpunkt etwa gleich groß ist. Im weiteren Reaktionsverlauf verlangsamt sich dann die Hexachlorethanbildung bei Abwesenheit von Sauerstoff jedoch deutlich: Nach 3, 4 und 5 Stunden werden nur noch Hexachlorbutadienumsätze von 21, 25 und 27 % beobachtet, wobei die Selektivität der Hexachlorethanbildung konstant bei 81 % liegt.

Patentansprüche

1. Verfahren zur Herstellung von Hexachlorethan durch Umsetzung von gasförmigem Chlor mit flüssigem Hexachlorbutadien,
dadurch gekennzeichnet,
daß die Chlorierung in Gegenwart von Sauerstoff durchgeführt wird.

2. Verfahren nach Anspruch 1,
dadurch gekennzeichnet,
daß der zur Reaktion benötigte Sauerstoff dem Chlorstrom beigemischt wird.

3. Verfahren nach Anspruch 1 und 2,
dadurch gekennzeichnet,
daß als Sauerstofflieferant Luft eingesetzt wird.

4. Verfahren nach Anspruch 1 bis 3,
dadurch gekennzeichnet,
daß der Sauerstoffgehalt des eingesetzten Chlor-Sauerstoff-Stromes zwischen 0,1 und 10 Vol.-% liegt.

5. Verfahren nach Anspruch 1 bis 4,
dadurch gekennzeichnet,
daß der Druck des Reaktionssystems zwischen 1 und 50 bar (abs.) liegt.

6. Verfahren nach Anspruch 1 bis 4,
dadurch gekennzeichnet,
daß der Reaktionsdruck bei 4 bis 10 bar (abs.) liegt.

7. Verfahren nach Anspruch 1 bis 6,
dadurch gekennzeichnet,
daß die Reaktionstemperatur zwischen 100 °C und der Siedetemperatur der flüssigen Phase bei dem jeweils festgelegten Druck liegt.

8. Verfahren nach Anspruch 1 bis 6,
dadurch gekennzeichnet,
daß die Reaktionstemperatur zwischen 140 und 180 °C liegt.

Claims

1. Process for the production of hexachloroethane by reacting gaseous chloring with liquid hexachlorobutadiene, characterized in that chlorination is performed in the presence of oxygen.

2. Process according to Claim 1, characterized in that the oxygen required for the reaction is mixed with the stream of chlorine.

3. Process according to Claim 1 and 2, characterized in that air is used as the oxygen source.

4. Process according to Claims 1 to 3, characterized in that the oxygen content of the chlorine-oxygen stream used is between 0.1 and 10% by volume.

5. Process according to Claims 1 to 4, characterized in that the pressure of the reaction system is between 1 and 50 bar (abs.).

6. Process according to Claims 1 to 4, characterized in that the reaction pressure is 4 to 10 bar (abs.).

7. Process according to Claims 1 to 6, characterized in that the reaction temperature is between 100°C and the boiling point of the liquid phase at the pressure established at the time.

8. Process according to Claims 1 to 6, characterized in that the reaction temperature is between 140 and 180°C.

Revendications

1. Procédé pour la préparation d'hexachloréthane par réaction de chlore gazeux avec de l'hexachlorobutadiène liquide, caractérisé en ce que la chloration est effectuée en présence d'oxygène.

2. Procédé selon la revendication 1, caractérisé en ce que l'oxygène requis pour la réaction est ajouté au courant de chlore.

3. Procédé selon la revendication 1 ou 2, caractérisé en ce que l'on introduit de l'air en tant qu'agent fournissant de l'oxygène.

4. Procédé selon l'une quelconque des revendications 1 à 3, caractérisé en ce que la teneur en oxygène du courant de chlore-oxygène introduit est comprise entre 0,1 et 10% en volume.

5. Procédé selon l'une quelconque des revendications 1 à 4, caractérisé en ce que la pression du système réactionnel est comprise entre 1 et 50 bars (absolue).

6. Procédé selon l'une quelconque des revendications 1 à 4, caractérisé en ce que la pression de la réaction est de 4 à 10 bars (absolue).

7. Procédé selon l'une quelconque des revendications 1 à 6, caractérisé en ce que la température de la réaction est située entre 100°C et la température d'ébullition de la phase liquide, à la pression établie dans chaque cas.

8. Procédé selon l'une quelconque des revendications 1 à 6, caractérisé en ce que la température de la réaction est comprise entre 140 et 180°C.